# EUROPÄISCHE PATENTSCHRIFT

(11) **EP 1 140 013 B1**
(45) Veröffentlichungstag und Bekanntmachung des Hinweises auf die Patenterteilung: **17.09.2003**
(21) Anmeldenummer: 99966965.8
(22) Anmeldetag: 14.12.1999
(51) Int. Cl.: A61K 9/00, A61P 1/04

(54) **SCHÄUMENDE ANTACIDA-SUSPENSIONSTABLETTEN**
FOAMING ANTACID SUSPENSION TABLETS
COMPRIMES ANTIACIDES GENERANT DES SUSPENSIONS MOUSSANTES

(30) Priorität: 21.12.1998 DE 19859231
(43) Veröffentlichungstag der Anmeldung: 10.10.2001
(73) Patentinhaber: Dr. Regenold GmbH, 79410 Badenweiler (DE)
(72) Erfinder: BAUER, Kurt, H., D-79112 Freiburg (DE)
(74) Vertreter: Albrecht, Thomas, Dr.
(86) Internationale Anmeldenummer: EP9909934
(87) Internationale Veröffentlichungsnummer: WO00037043

(56) Entgegenhaltungen:
- US-A- 4 613 497
- US-A- 4 716 033
- US-A- 4 783 331
- US-A- 5 330 760

## Beschreibung

Die Erfindung betrifft Zubereitungen, die einen oder mehrere säurebindende Wirkstoffe (Antacidum) enthalten, sowie ein Verfahren zur Herstellung dieser Zubereitungen und ihre Verwendung als Arzneimittel zur Regulation der Hyperacidität des Magens.

Zur Behandlung einer Reihe von akuten und chronischen Magen-Darm-Beschwerden hat sich die Therapie mit Antacida hervorragend bewährt. Insbesondere werden Antacida zur symptomatischen Therapie von Ulcus duodeni und Ulcus ventriculi sowie bei Sodbrennen und säurebedingten Magenbeschwerden, wie hyperacider Gastritis, verwendet. Die Wirkung der Antacida beruht dabei im wesentlichen darauf, daß die Magensalzsäure teilweise abgepuffert wird, so daß es zu einer Erhöhung des pH-Wertes des Magens von 1 bis 2 auf 3 bis 4 kommt. Durch diese Erhöhung des pH-Wertes können die für eine Hyperacidität typischen Beschwerden, wie z.B. Völlegefühl oder Sodbrennen, gemindert oder sogar behoben werden. Bisher werden Antacida in Pulverform, als normale Tabletten, vorwiegend als Kautabletten oder als Suspensionen in Flaschen oder abgepackt in Sachets vertrieben. In der deutschen Patentanmeldung DE 44 24 676 werden zudem Antacida-Brausetabletten beschrieben.

US-A-4613497 offenbart eine Antacida-Zusammensetzung, enthaltend säurebindende Wirkstoffe, eine Brausemischung, ein Polysaccharid-Gummi und ein gelbildendes Salz.

Die bekannten Kautabletten lassen sich sehr leicht und einfach dosieren, besitzen jedoch den Nachteil, daß die Patienten diese nach dem Zerkauen als sandig und somit als unangenehm empfinden. Im Gegensatz dazu fühlen sich die bekannten fein dispergierten Suspensionsarzneiformen nicht sandig an, sind gut verträglich und zeichnen sich zudem durch einen raschen und deutlich spürbaren Wirkungseintritt und Effekt aus. Nachteilig an diesen Suspensionsarzneiformen ist jedoch die Tatsache, daß insbesondere ältere Patienten Schwierigkeiten haben, die Suspensionen vollständig und ohne größere Schwierigkeiten aus den Sachet-Packungen zu entnehmen. Demgegenüber ist die Dosierung und Verabreichung von Brausetabletten einfacher, jedoch erfordern die bisherigen Brausetabletten zur Einnahme ein Glas Wasser, in dem sich die Brausetabletten unter Aufbrausen zu einer flüssigen trinkbaren Suspension auflösen. Dadurch ist eine Einnahme des Medikamentes nicht bei jeder Gelegenheit möglich. Somit haben alle bisher verfügbaren Zubereitungsformen von Antacida gewisse Nachteile bei der Verabreichung.

Der Erfindung liegt die Aufgabe zugrunde, eine Antacida-Zubereitung bereitzustellen, mit der eine gut und rasch wirkende Suspension in möglichst gleich feiner Dispersität hergestellt werden kann, beispielsweise bevorzugt durch Kauen und Einspeicheln, wobei die Antacida-Zubereitung gleichzeitig besser, sicherer und unkomplizierter zu applizieren ist als die aus dem Stand der Technik bekannten Antacida-Formulierungen.

Diese Aufgabe wird durch die in den Patentansprüchen aufgeführten Merkmale gelöst.

Insbesondere betrifft die Erfindung Antacidum-Kautabletten, die beim Kauen im Mund ein Schaumgel entwickeln. Derartige Tabletten können so hergestellt werden, daß sie auch von älteren Patienten noch leicht gekaut werden können. Kautabletten ermöglichen eine zufriedenstellende Dosierung des Wirkstoffes, sind leicht aus ihren Verpackungen zu entnehmen und erfordern zur Einnahme keine Flüssigkeit. Die neuartigen erfindungsgemäßen Tabletten entwickeln beim Kauen und Vermischen mit dem Speichel ein schaumiges Suspensionsgel, das im Mund als angenehm empfunden wird und das problemlos geschluckt werden kann. Im Magen führt dieses hochdisperse Schaumgel zu einer überraschend guten Pufferung.

Die Erfindung betrifft eine Antacida-Zubereitung, die die. folgenden Bestandteile umfaßt:
(i) einen säurebindenden Wirkstoff (Antacidum) oder ein Gemisch solcher Wirkstoffe,
(ii) eine CO₂ freisetzende Brausemischung,
iii) ein oder mehrere polymere Tenside ausgewählt aus Poloxameren als Schaumbildner,
iv) ein von der Komponente (iii) verschiedenes quellendes und gelbildendes Polymer oder ein Gemisch solcher Polymere und
(v) gegebenenfalls übliche Hilfsstoffe.

Säurebindende Wirkstoffe für Antacida-Zubereitungen sind dem Fachmann bekannt. Insbesondere kommen als säurebindende Wirkstoffe Magnesiumhydroxid, Magnesiumoxid, Magnesiumcarbonat, Magnesiumsilicat, Aluminiumhydroxid, Aluminiumphosphat und Magnesium-Aluminiumsilicat oder Gemische davon in Frage. Bevorzugt sind Aluminiumhydroxid, Magnesiumhydroxid, Hydrotalcit oder Magaldrat, ein Magnesium-Aluminiumsilicat. Besonders bevorzugt ist Magaldrat. Dem Fachmann ist zum Beispiel aus der DE 44 24 676 bekannt, daß aluminiumhaltige Arzneimittel unter bestimmten Bedingungen toxisch wirken können, insbesondere wenn die Aluminiumverbindung gemeinsam mit bestimmten Säuren, wie z.B. Citronensäure, verabreicht wird. Bei der Auswahl der oben genannten möglichen säurebindenden Wirkstoffe wird sich der Fachmann somit nach der Gesamtzusammensetzung der Antacida-Zubereitung richten und beispielsweise Aluminiumverbindungen nicht verwenden bzw. in geringerer Dosierung verwenden, wenn ein anderer Bestandteil der erfindungsgemäßen Zubereitung Citronensäure ist, oder aber der Fachmann wird umgekehrt Citronensäure nicht oder nur in geringer Menge verwenden, wenn die Zubereitung eine Aluminiumverbindung enthalten soll.

Die säurebindenden Wirkstoffe werden erfindungsgemäß in einer Konzentration von 20 bis 80 Gew.-%, bevorzugt 40 bis 75 Gew.-%, bevorzugter 50 bis 70 Gew.-%, bezogen auf das Gesamttrockengewicht der Zubereitung, eingesetzt.

Die verwendete CO₂ entwickelnde Brausemischung umfaßt einen sauren Bestandteil sowie einen basischen Bestandteil. Als saure Bestandteile der CO₂ entwickelnden Brausemischung kommen beispielsweise Citronensäure, Weinsäure, Adipinsäure, Ascorbinsäure, Äpfelsäure, Fumarsäure und Maleinsäure wie auch saure Salze, beispielsweise Kaliumbitartrat, primäres Natriumphosphat, primäres Natriumcitrat, primäres Natriumtartrat oder Gemische davon in Frage. Bevorzugt sind Citronensäure, Weinsäure, Adipinsäure und primäres Natriumcitrat, besonders bevorzugt sind Citronensäure, Weinsäure und primäres Natriumcitrat. Wie bereits ausgeführt, wird der Fachmann bei der Wahl der als sauren Bestandteil der CO₂ entwickelnden Brausemischung zu verwendenden Säure auf die Gesamtformulierung der Zubereitung achten und insbesondere die Verwendung bestimmter Säuren vermeiden, wenn dadurch eine erhöhte Toxizität der säurebindenden Wirkstoffe zu befürchten ist.

Weitere geeignete Säurekomponenten der CO₂ entwickelnden Brausemischung sind saure Salze basischer Aminosäuren, wie Glycin, Alanin, Valin, Ornithin oder Lysin.

Als basische Bestandteile der CO₂ entwickelnden Brausemischung kommen im allgemeinen CO₂ entwickelnde Verbindungen in Frage, insbesondere Natriumhydrogencarbonat, Natriumcarbonat, Calciumcarbonat oder Gemische davon.

Das Verhältnis des sauren Bestandteils der CO₂ entwickelnden Brausemischung zum basischen Bestandteil der CO₂ entwickelnden Brausemischung ist in der Regel äquimolekular/stöchiometrisch. Da jedoch auch die antacid wirkenden Arzneistoffe basisch reagieren, kann es aus Pufferungs- oder Geschmacksgründen (Einstellung eines optimalen pH-Wertes) erforderlich sein, die Säurekomponente überzudosieren.

Der Anteil der CO₂ entwickelnden Brausemischungen am Gesamttrockengewicht der Antacida-Zubereitung beträgt 5 bis 50 Gew.-%, vorzugsweise 7,5 bis 25 Gew.-%, am meisten bevorzugt 10 bis 20 Gew.-%, Zur Formulierung als Antacidum-Kautablette, die beim Kauen im Mund ein Schaumgel entwickelt, werden die Bestandteile der Brausemischung bevorzugt so stöchiometrisch berechnet sein, daß das mit den übrigen Bestandteilen und dem Speichel im Mund entstehende Schaum-Suspensionsgel schwach alkalisch reagiert. Unter schwach alkalisch wird ein pH von 7 bis 9, bevorzugt 7 bis 8 verstanden.

Ist die Antacida-Zubereitung als Kautablette formuliert, so sind die CO₂ entwickelnden Brausemischungen in den erfindungsgemäßen schäumenden Suspensionstabletten grundsätzlich deutlich geringer dosiert als in Brausetabletten. Sie sollen nämlich keine flüssige Suspension ergeben, sondern vielmehr ein leicht schluckbares Schaumgel, welches die Wirkstoffe im Magen rasch und feindispers über große Flächen der Magenschleimhaut freisetzt.

Als polymeres, schaumbildendes Tensid ("Schaumbildner") werden für die erfindungsgemäßen Zubereitungen gut verträgliche polymere Tenside ausgewählt aus Poloxameren (Blockcopolymere aus Ethylenoxid und Propylenoxid) verwendet.

Besonders bevorzugt sind Pluronic® F 68 oder Pluronic® F 127 (BASF AG Ludwigshafen/Rh. oder Wyandotte Chem. Corp., Biddle, Wyandotte, Michigan), die nicht oder nur in geringem Maße hämolytisch (blutkörperchenzerstörend) sind. Der Gehalt der formbildenden polymeren Tenside in der Zubereitung beträgt 0,5 bis 30 Gew.-%, vorzugsweise 1 bis 20 Gew.-%, am meisten bevorzugt 3 bis 10 Gew-%, bezogen auf das Gesamttrockengewicht der Zubereitung.

Als quellendes und gelbildendes Polymer kommen vor allem Polysaccharide und Polysaccharid-Derivate oder aber Polyacrylsäuren in Frage. Beispiele für Polysaccharide und Polysaccharid-Derivate sind Traganth, Galactomannan, Celluloseether, beispielsweise Methylcellulose und gemischte Celluloseether, wie Hydroxypropyl- oder Hydroxybutylmethylcellulose. Unter dem Markennamen Methocel® werden derartige Methylcelluloseund gemischte Celluloseether von Dow Chemical vertrieben. Beispiele für Polyacrylsäuren, die erfindungsgemäß verwendet werden können, sind Carbopol® oder Eudispert® (pharmazeutisch gebräuchliche bzw. zugelassene Carbapol- und Eudispert-Typen). Der Anteil der quellenden, gelbildenden Polymere am Gesamttrockengewicht der Zubereitung beträgt 3 bis 30%, vorzugsweise 5 bis 15%, am bevorzugtesten 7,5 bis 10%.

Die Antacida-Zubereitung enthält in der Regel eine Restfeuchte von nicht mehr als 6%, bevorzugt nicht mehr als 3%, am meisten bevorzugt nicht mehr als 1%, bezogen auf das Gesamtgewicht der Zubereitung.

Die Antacida-Zubereitung kann zudem noch übliche Tablettenhilfsstoffe enthalten, wie beispielsweise Zuckeralkohole oder Zucker (Glucose, Lactose, etc.) als Füllmittel, Süßmittel, Aromastoffe, sowie Gleit-, Formtrenn- und Fließregulierungsmittel.

Die erfindungsgemäße Zubereitung eignet sich zur Verwendung als Antacidum, also als Arzneimittel zur Regulation der Hyperacidität des Magens. Die erfindungsgemäßen Zubereitungen können dabei insbesondere in Form einer Kautablette formuliert sein. Daneben sind allerdings auch noch andere Verabreichungsformen möglich, wie z.B. die Verabreichung in Form eines Pulvers oder Granulats.

Durch die Erfindung wird weiterhin ein Verfahren zur Herstellung einer wie oben definierten Zubereitung bereitgestellt, welches die folgenden Stufen umfaßt:
(i) Homogenes Mischen eines säurebindenden Wirkstoffes oder Gemischen davon mit dem basischen Bestandteil der CO₂ freisetzenden Brausemischung sowie gegebenenfalls mit einem oder mehreren quellenden, gelbildenden Polymeren,
ii) Herstellung einer wässrigen Lösung/Suspension, die einen oder mehrere Schaumbildner ausgewählt aus Poloxameren sowie gegebenenfalls ein quellendes, gelbildendes Polymer oder Gemische solcher Polymere enthält,
(iii) Vereinigen und Mischen des unter (i) erhaltenen homogenen Gemisches mit der unter (ii) erhaltenen Suspension/Lösung, Feuchtgranulieren des Gemisches sowie Trocknen und Sieben des erhaltenen Granulates,
(iv) Herstellung einer homogenen Trockenmischung aus dem sauren Bestandteil der CO₂ entwickelnden Brausemischung und gegebenenfalls einem oder mehreren quellenden, gelbildenden Polymeren,
(v) Zumischen der in (iv) erhaltenen Mischung zu dem unter (iii) erhaltenen Granulat und gegebenenfalls Pressen des so erhaltenen Granulates zu Tabletten,
wobei das quellende, gelbildende Polymer bei mindestens einer der Stufen (i), (ii) oder (iv) homogen zugemischt wird und
wobei bei den einzelnen Verfahrensstufen gegebenenfalls noch übliche Tablettenhilfsstoffe zugegeben werden können.

In der ersten Stufe (i) des erfindungsgemäßen Verfahrens wird der säurebindende Wirkstoff oder ein Gemisch dieser Wirkstoffe mit dem basischen Bestandteil der CO₂ freisetzenden Brausemischung homogen gemischt. Gegebenenfalls kann bereits zu dieser Stufe ein quellendes, gelbildendes Polymer oder ein Gemisch mehrerer dieser Polymere zugegeben werden. Die homogene Mischung kann vorzugsweise durch einen Intensivmischer oder -mischkneter erfolgen.

Parallel dazu wird als Stufe (ii) eine wäßrige Lösung des Schaumbildners also beispielsweise eine wäßrige Lösung von Pluronic® F 68 oder Pluronic® F 127 hergestellt. Als wäßriges Lösungsmittel kann Wasser oder eine wäßrige Tensid- oder Süßmittellösung, beispielsweise Poloxamer- oder Saccharinlösung, verwendet werden. Es wird mindestens soviel wäßrige Lösung verwendet, daß der zu verwendende Schaumbildner bzw. die zu verwendenden Schaumbildner sich gerade noch lösen. Bevorzugt ist die eingesetzte Wassermenge gerade so hoch, daß die betreffende Masse feuchtplastisch und ausreichend gut granulierbar wird. Der wäßrigen Lösung des Schaumbildners können gegebenenfalls ein quellendes, gelbildendes Polymer oder Gemische davon zugegeben werden. Dabei wird durch Rühren eine homogene Suspension hergestellt.

In einer dritten Stufe (iii) wird das in Stufe (i) erhaltene homogene Gemisch aus säurebindendem Wirkstoff oder Gemischen davon und dem basischen Bestandteil der CO₂ freisetzenden Brausemischung, das gegebenenfalls zusätzlich ein oder mehrere quellende gelbildende Polymere enthält, mit der in Stufe (ii) erhaltenen wäßrigen Lösung des Schaumbildners bzw. der wäßrigen Suspension des Schaumbildners und des quellenden gelbildenden Polymeren oder Gemischen davon vereinigt und gemischt. Zum Mischen dieser Bestandteile verwendet man einen Mischer oder Kneter mit Rühr- oder Knetwerkzeugen. Nach dem Vereinigen und Mischen wird das erhaltene Gemisch feucht granuliert. Das Feuchtgranulieren erfolgt durch Feuchtigkeitszugabe bis zur feuchtplastischen und damit granulierbaren Konsistenz. Nach der Feuchtgranulation wird das erhaltene Granulat getrocknet. Dabei wird die Trocknung bis zu einem Restfeuchtegehalt von 0,1 bis 6,0%, vorzugsweise 1 bis 3%, am meisten bevorzugt < 1,0% getrocknet. Als Trockner können übliche Trocknungsvorrichtungen, wie Trockenschränke und Wirbelschichttrockner,eingesetzt werden. Geeignete Trockner werden beispielsweise in Bauer, Frömming, Führer, "Pharmazeutische Technologie", G. Fischer Verlag, Stuttgart, Jena, 5. Auflage, 1997, Seiten 123 bis 130 beschrieben. Bevorzugt wird die Vortrocknung bei Temperaturen von 30 bis 70°C, bevorzugter 40 bis 60°C, am bevorzugtesten 45 bis 50°C durchgeführt.

Anschließend wird das getrocknete Granulat gesiebt. Als Siebe kommen übliche Siebe, die beispielsweise in Bauer, Frömming, Führer, "Pharmazeutische Technologie", G. Fischer Verlag, Stuttgart, Jena, 5. Auflage, 1997, Seite 108 (Siebe) beschrieben werden, in Betracht. Geeignete Siebe haben eine Maschenweite von 0,1 bis 3,0 mm, vorzugsweise 0,5 bis 1,5 mm. Das Sieben erfolgt bei Normaldruck oder gegebenenfalls unter Anwendung von Überdruck. Die geeigneten Siebmaschenweiten hängen von der gewünschten bzw. geforderten Dispersität oder Homogenität ab.

In der vierten Stufe (iv) wird eine homogene Trockenmischung aus dem sauren Bestandteil der CO₂ entwickelnden Brausemischung und gegebenenfalls einem der mehreren quellenden Gelpolymeren hergestellt. Zur Herstellung dieser homogenen Trokkenmischung kann die gleiche Einrichtung verwendet werden, die für die erste Stufe des Verfahrens beschrieben wurde.

In der nächsten Stufe (v) wird die in Stufe (iv) erhaltene Mischung aus dem sauren Bestandteil der CO₂ entwickelnden Brausemischung und gegebenenfalls einem oder mehreren gelbildenden Polymeren dem in der dritten Stufe erhaltenen Granulat zugemischt. Zur Mischung kann beispielsweise die gleiche Apparatur wie die für die erste Stufe beschriebene verwendet werden. Das so erhaltene Granulat kann dann beispielsweise zu kaubaren Tabletten verpreßt werden. Bevorzugt werden die Tabletten so hart verpreßt, daß sie gut verpackungsfähig und noch gut kaubar sind. Als Tablettierungseinrichtungen kommen die bekannten Rundlauf- und Exzentertablettenpressen in Frage. Die Härte der Kau-Schaumgel-Tabletten soll vorzugsweise bei 4 bis 7 kp liegen.

Bei dem erfindungsgemäßen Verfahren können bei jeder der Stufen nach Bedarf übliche Tablettenhilfsstoffe, wie z.B. die oben definierten Hilfsstoffe, zugegeben werden. Das quellende, gelbildende Polymer muß bei mindestens einer der Stufen (i), (ii) oder (iv) zugemischt werden. Bei einer besonders bevorzugten Ausführungsform wird das quellende, gelbildende Polymer bei den Stufen (ii) und (iv) zugegeben. Bei einer anderen bevorzugten Ausführungsform wird das quellende, gelbildende Polymer lediglich bei der Stufe (i) zugegeben. Bei einer weiteren bevorzugten Ausführungsform wird das quellende, gelbildende Polymer lediglich bei der Stufe (iv) zugegeben. Es ist dabei darauf zu achten, daß sauer und basisch reagierende Stoffe so oder in einem Zustand vermischt werden, daß sie nicht vorzeitig oder unbeabsichtigt reagieren können. Dies geschieht beispielsweise dadurch, daß sie getrennt granuliert und nur in trockenem, nicht reaktionsfähigem Zustand vermischt werden.

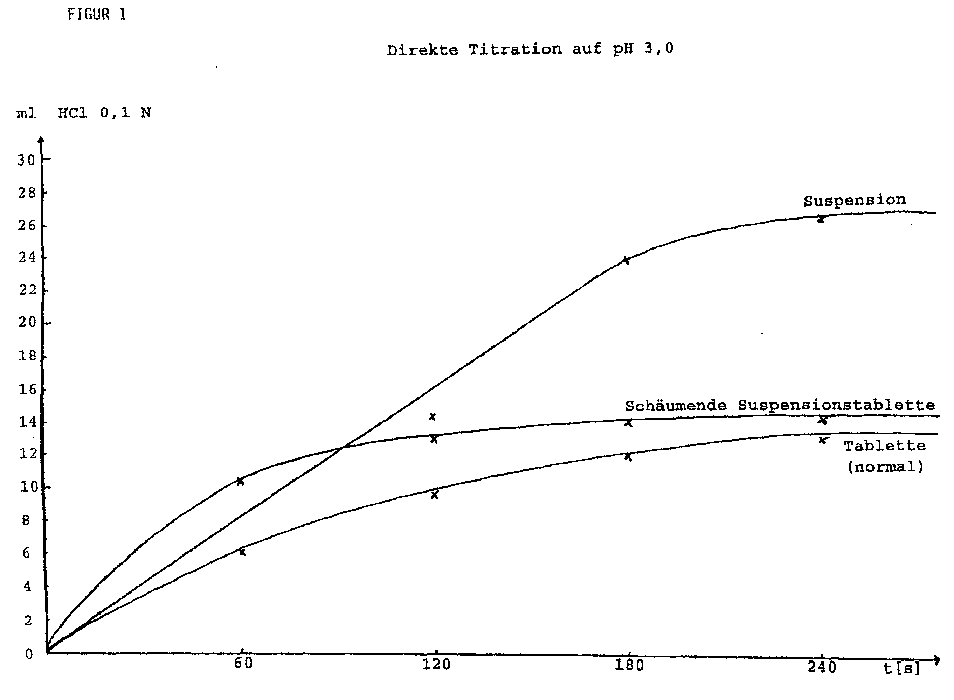

Die nach dem erfindungsgemäßen Verfahren hergestellten Zubereitungen können in Form einer Kautablette formuliert werden. Im Magen führt das nach Kauen im Mund in Verbindung mit Speichel entstehende, hochdisperse Schaumgel, wie die Figur 1 zeigt, zu einer überraschend raschen Pufferung. Die Pufferkapazitäten liegen zwischen denen einer normalen Aluminiumhydroxid-Gel/Magnesiumhydroxid-Gel-Suspension und einer normalen Aluminiumhydroxid-Gel/Magnesiumhydroxid-Gel-Tablette. Die Erfindung wird anhand der Figur 1 und den folgenden Beispielen näher erläutert werden:
Figur 1 zeigt die Bestimmung der Pufferkapazität durch die direkte Titration von ausgewählten Antacidum-Zubereitungen mit 0,1 N HCl in ml/sec.

### BEISPIELE

### Beispiel 1

40,0 g Aluminiumoxid-Trockengel, 40,0 g Magnesiumoxid-Trokkengel, 10,0 g Natriumhydrogencarbonat und 10,0 g schweres basisches Magnesiumcarbonat werden homogen gemischt. 0,1 g Saccharin-Natrium und 30,0 g Poloxamer 188 (Pluronic® F 68) werden in 50 bis 60 ml warmem Wasser gelöst, und anschließend werden 3,0 g Methocel in dieser Lösung knöllchenfrei suspendiert. Beim Abkühlen löst sich das Methocel praktisch quantitativ auf. Die Mischung der Wirkstoffe mit den Carbonaten wird mit der erhaltenen Lösung feuchtgranuliert und bei 45°C getrocknet und gesiebt. Anschließend werden 12,0 g Methocel, 15,0 g Citronensäure wasserfrei, 18,2 g Mannitol, 1,5 g Magnesiumstearat und 0,2 g Aroma homogen trockengemischt und diese Mischung als äußere Phase dem vorher erhaltenen Granulat zugemischt. Das Granulat wird zu Tabletten mit 1800,0 mg Bruttogewicht und einem Durchmesser von 16 mm gepreßt.

### Beispiel 2

40,0 g Aluminiumhydroxid-Gel, 40,0 g Magnesiumhydroxid-Gel, 20,0 g Natriumhydrogencarbonat und 15,0 g Natrium-Carboxymethylcellulose werden homogen gemischt. Diese Mischung wird mit einer Lösung aus 0,1 g Saccharin-Natrium und 40,0 g Poloxamer 407 (Pluronic® F 127) in 70 bis 80 ml Wasser feuchtgranuliert, bei 40°C getrocknet und gesiebt. Anschließend werden 20,0 g Citronensäure (wasserfrei/Anhydrat), 18,1 g Lactose (DAB/EuAB), 1,5 g Magnesiumstearat (DAB/EuAB) und 0,3 g Waldhimbeeraroma als äußere Phase dazugemischt. Das Granulat wird zu Tabletten mit 18 mm Durchmesser und einem Bruttogewicht von 1950,0 mg gepreßt.

### Beispiel 3

750,0 g Magaldrat, 150,0 g Calciumcarbonat und 150,525 g Xylitol werden homogen gemischt und gesiebt. Die resultierende Mischung wird mit einer Lösung aus 0,975 g Saccharin-Natrium, 9,0 g Cyclamat-Natrium und 90,0 g Pluronic® F 68 in 300,0 bis 450,0 ml Wasser in bekannter Weise angeknetet, feuchtgranuliert (Sieb 2 mm) und bei 60°C getrocknet (Restfeuchtigkeit 4 bis 6%). Nach dem Trocknen werden diesem Granulat als äußere Phase 105,0 g Citronensäure-Anhydrat, 9,75 g Magnesiumstearat, 25,0 g Polyacrylsäure und 9,75 g Orangenaroma zugemischt, und das Granulat wird in bekannter Weise tablettiert.

### Beispiel 4

750,0 g Magaldrat Codried, 250,0 g Natriumcarbonat/Natriumhydrogencarbonat und 145,025 g Glucose werden homogen gemischt und gesiebt. Die resultierende Mischung wird mit einer Lösung aus 0,975 g Saccharin-Natrium, 9,0 g Cyclamat-Natrium und 120,0 g Pluronic® F 127 in 300,0 bis 400,0 ml Wasser in bekannter Weise angeknetet, feuchtgranuliert (Sieb 2 mm) und bei 45°C getrocknet (Restfeuchtigkeit 5 bis 8%). Nach dem Trocknen werden diesem Granulat als äußere Phase 105,0 g Weinsäure, 12,0 g Magnesiumstearat, 8,0 g Aroma und 100,0 g Natrium-Carboxymethylstärke zugemischt,und das Granulat wird in bekannter Weise tablettiert.

### Beispiel 5

500,0 g Hydrotalcit, 120,0 g Natriumcarbonat und 103,2 g Mannitol werden homogen gemischt und gesiebt (Sieb 1 mm). Die resultierende Mischung wird mit einer Lösung aus 0,8 g Saccharin-Natrium, 8,0 g Cyclamat-Natrium und 80,0 g Pluronic® F 127 in 250,0 bis 300,0 ml Wasser in bekannter Weise angeknetet, feuchtgranuliert (Sieb 2 mm) und bei 60°C getrocknet (Restfeuchtigkeit 4 bis 6%). Nach dem Trocknen werden diesem Granulat als äußere Phase 90,0 g Citronensäure-Anhydrat/Natriumcitrat primär, 10,0 g Calciumarachinat und 80,0 g Hydropropylcellulose und 8,0 g Aroma zugemischt,und das Granulat wird in bekannter Weise tablettiert. Diese Formulierung kann zur Herstellung von Tabletten mit 500,0 mg oder 1000,0 mg verwendet werden. Es sollten Tablettendurchmesser gewählt werden, die gewährleisten, daß die Tabletten nicht zu dick und damit schlecht kaubar werden.

### Beispiel 6

### Bestimmung der Pufferkapazität der erfindungsgemäßen schäumenden Antacida-Suspensionstabletten

Es wurde die Pufferkapazität einer als Handelsware erhältlichen Antacida-Suspension, einer als Handelsware erhältlichen Antacida-Tablette und einer erfindungsgemäßen schäumenden Antacida-Suspensionstablette miteinander verglichen. Alle Zubereitungen enthielten die gleiche Menge an Wirkstoff. Die Pufferkapazität wurde durch direkte Titration der genannten Zubereitungen mit 0,1 N HCl bestimmt. Das Experiment zeigt, daß die erfindungsgemäßen schäumenden Antacida-Suspensionstabletten Säure deutlich besser abpuffern als herkömmliche Tabletten. Herkömmliche flüssige Suspensionen zeigen insgesamt die höchste Pufferkapazität, wobei die Suspensionen anfänglich langsamer abpuffern als die erfindungsgemäßen schäumenden Antacida-Suspensionstabletten. Die Ergebnisse dieses Experimentes sind in Figur 1 graphisch wiedergegeben.

## Patentansprüche

1. Antacida-Zubereitung, umfassend die folgenden Bestandteile:
(i) einen säurebindenden Wirkstoff (Antacidum) oder ein Gemisch solcher Wirkstoffe,
(ii) eine CO₂ freisetzende Brausemischung,
iii) ein oder mehrere polymere Tenside ausgewählt aus Poloxameren als Schaumbildner,
iv) ein von der Komponente (iii) verschiedenes quellendes und gelbildendes Polymer oder ein Gemisch solcher Polymere und
(v) gegebenenfalls übliche Hilfsstoffe.

2. Antacida-Zubereitung nach Anspruch 1, **dadurch gekennzeichnet, daß** die säurebindenden Wirkstoffe ausgewählt sind aus Magnesiumhydroxid, Magnesiumoxid, Magnesiumcarbonat, Magnesiumsilicat, Aluminiumbydroxid, Aluminiumphosphat und Magnesium-Aluminiumsilicat oder Gemischen davon.

3. Antacida-Zubereitung nach einem der Ansprüche 1 bis 2, **dadurch gekennzeichnet, daß** die CO₂ freisetzende Brausemischung einen sauren Bestandteil sowie einen basischen Bestandteil enthält.

4. Antacida-Zubereitung nach Anspruch 3, **dadurch gekennzeichnet, daß** die sauren Bestandteile der CO₂ entwickelnden Brausemischungen ausgewählt sind aus Citronensäure, Weinsäure, Adipinsäure, Ascorbinsäure, Äpfelsäure, Fumarsäure, Maleinsäure, Kaliumbitartrat, primärem Natriümphosphat, primärem Natriumcitrat, primärem Natriumtartrat oder sauren Salzen basischer Aminosäuren oder Gemischen davon und die basischen Bestandteile der CO₂ entwickelnden Brausemischung ausgewählt sind aus Natriumhydrogencarbonat, Natriumcarbonat, Calciumcarbonat oder Gemischen davon.

5. Antacida-Zubereitung nach einem der Ansprüche 1 bis 4, **dadurch gekennzeichnet, daß** die stark quellenden und gelbildenden Polymere ausgewählt sind aus Polysacchariden, Polysaccharid-Derivaten und Polyacrylsäuren.

6. Antacida-Zubereitung nach einem der Ansprüche 1 bis 5, **dadurch gekennzeichnet, daß** die Konzentration der säurebindenden Wirkstoffe 20 bis 80 Gew.-%, der CO₂ freisetzenden Brausemischung 5 bis 50 Gew.-%, des polymeren schaumbildenden Tensids 0,5 bis 30 Gew.-% und des quellenden und gelbildenden Polymers 3 bis 30 Gew.-%, bezogen auf das Gesamttrockengewicht der Zubereitung, beträgt.

7. Antacida-Zubereitung nach einem der Ansprüche 1 bis 6 zur Verwendung als Arzneimittel zur Regulation der Hyperacidität des Magens.

8. Antacida-Zubereitung nach Anspruch 7 **dadurch gekennzeichnet, daß** die Zubereitung in Form einer Kautablette formuliert ist.

9. Antacida-Verfahren zur Herstellung einer Antacids-Zubereitung nach einem der Ansprüche 1 bis 8 umfassend die folgenden Stufen:
(i) Homogenes Mischen eines säurebindenden Wirkstoffes oder Gemischen davon mit dem basischen Bestandteil der CO₂ freisetzenden Brausemischung sowie gegebenenfalls mit einem oder mehreren quellenden, gelbildenden Polymeren,
ii) Herstellung einer wässrigen Lösung/Suspension, die einen oder mehrere Schaumbildner ausgewählt aus Poloxameren sowie gegebenenfalls ein quellendes, gelbildendes Polymer oder Gemische solcher Polymere enthält,
(iii) Vereinigen und Mischen des unter (i) erhaltenen homogenen Gemisches mit der unter (ii) erhaltenen Suspension/Lösung, Feuchtgranulieren des Gemisches sowie Trocknen und Sieben des erhaltenen Granulates,
(iv) Herstellung einer homogenen Trockenmischung aus dem sauren Bestandteil der CO₂ entwickelnden Brausemischung und gegebenenfalls einem oder mehreren quellenden, gelbildenden Polymeren,
(v) Zumischen der in (iv) erhaltenen Mischung zu dem unter (iii) erhaltenen Granulat und gegebenenfalls Pressen des so erhaltenen Granulates zu Tabletten,
wobei das quellende, gelbildende Polymer bei mindestens einer der Stufen (i), (ii) oder (iv) homogen zugemischt wird und
wobei bei den einzelnen Verfahrensstufen gegebenenfalls noch übliche Tablettenhilfsstoffe zugegeben werden können.

10. Verfahren nach Anspruch 9, **dadurch gekennzeichnet, daß** die Tabletten so hart gepreßt werden, daß sie noch gut kaubar sind.

11. Verwendung einer Antacida-Zubereitung nach einem der Ansprüche 1 bis 8 oder einer Antacida-Zubereitung, die nach einem der Ansprüche 9 bis 10 bergestellt wurde, zur Herstellung eines Medikaments zur Regulation der Hyperacidität des Magensaftes.

## Claims

1. Antacid preparation comprising the following constituents:
(i) an acid-binding active constituent (antacid) or a mixture of such active constituents,
(ii) an effervescent mixture that releases CO₂,
(iii) one or more polymeric surfactants selected from poloxamers as foam-forming agent,
(iv) a swelling and gel-forming polymer different from the component (iii) or a mixture of such polymers, and
(v) optionally conventional auxiliary substances.

2. Antacid preparation according to claim 1,
**characterised in that** the acid-binding active constituents are selected from magnesium hydroxide, magnesium oxide, magnesium carbonate, magnesium silicate, aluminium hydroxide, aluminium phosphate and magnesium aluminium silicate or mixtures thereof.

3. Antacid preparation according to one of claims 1 and 2, **characterised in that** the effervescent mixture that releases CO₂ contains an acid constituent as well as a basic constituent.

4. Antacid preparation according to claim 3, **characterised in that** the acid constituents of the CO₂-developing effervescent mixtures are selected from citric acid, tartaric acid, adipic acid, ascorbic acid, malic acid, fumaric acid, maleic acid, potassium bitartrate, primary sodium phosphate, primary sodium citrate, primary sodium tartrate or acid salts of basic amino acids or mixtures thereof, and the basic constituents of the CO₂-developing effervescent mixture are selected from sodium hydrogen carbonate, sodium carbonate, calcium carbonate or mixtures thereof.

5. Antacid preparation according to one of claims 1 to 4, **characterised in that** the strongly swelling and gel-forming polymers are selected from polysaccharides, polysaccharide derivatives and polyacrylic acids.

6. Antacid preparation according to one of claims 1 to 5, **characterised in that** the concentration of the acid-binding active constituents is 20 to 80 wt.%, of the effervescent mixture, that releases CO₂ is 5 to 50 wt.%, of the polymeric foam-forming surfactant is 0.5 to 30 wt.%, and of the swelling and gel-forming polymer is 3 to 30 wt.%, referred to the total dry weight of the preparation.

7. Antacid preparation according to one of claims 1 to 6 for use as a medicament for regulating gastric hyperacidity.

8. Antacid preparation according to claim 7, **characterised in that** the preparation is formulated in the form of a chewable tablet.

9. Process for the production of an antacid preparation according to one of claims 1 to 8, comprising the following steps:
(i) homogeneous mixing of an acid-binding active constituent or mixtures thereof with the basic constituent of the effervescent mixture that releases CO₂, as well as optionally with one or more swelling, gel-forming polymers,
(ii) production of an aqueous solution/suspension containing one or more foam-forming agents selected from poloxamers, as well as optionally a swelling, gel-forming polymer or mixtures of such polymers,
(iii) combining and mixing of the homogeneous mixture obtained in (i) with the suspension/solution obtained in (ii), wet granulating of the mixture, as well as drying and screening of the granules obtained,
(iv) production of a homogeneous dry mixture from the acid constituent of the CO₂-developing effervescent mixture and optionally one or more swelling, gel-forming polymers,
(v) mixing of the mixture obtained in (iv) with the granules obtained in (iii) and optïonally pressing of the granules obtained into tablets,
wherein the swelling, gel-forming polymer is homogeneously mixed in in at least one of the stages (i), (ii) or (iv) and wherein conventional tablet auxiliary substances optionally can also be added in the individual process steps.

10. Process according to claim 9, **characterised in that** the tablets are compressed sufficiently hard so that they are still easily chewable.

11. Use of an antacid preparation according to one of claims 1 to 8 or an antacid preparation that has been produced according to one of claims 9 and 10, for the production of a medicament for regulating hyperacidity of the gastric juices.

## Revendications

1. Préparation d'antiacide comprenant les éléments constitutifs suivants :
(i) un principe actif liant les acides (antiacide) ou un mélange de principes actifs de ce genre,
(ii) un mélange effervescent libérant du CO₂,
(iii) un ou plusieurs agents tensio-actifs choisis parmi des poloxamères en tant qu'agents de moussage,
(iv) un polymère gonflant et gélifiant, différent du composant (iii), ou un mélange de polymères de ce genre, et
(v) le cas échéant des adjuvants usuels.

2. Préparation antiacide selon la revendication 1, **caractérisée en ce que** les principes actifs liant les acides sont choisis parmi l'hydroxyde de magnésium, l'oxyde de magnésium, le carbonate de magnésium, le silicate de magnésium, l'hydroxyde d'aluminium, le phosphate d'aluminium et l'aluminosilicate de magnésium ou des mélanges de ceux-ci.

3. Préparation antiacide selon l'une des revendications 1 à 2, **caractérisée en ce que** le mélange effervescent libérant du CO₂ contient un constituant acide ainsi qu'un constituant basique.

4. Préparation antiacide selon la revendication 3, **caractérisée en ce que** les constituants acides des mélanges effervescents dégageant du CO₂ sont choisis parmi l'acide citrique, l'acide tartrique, l'acide adipique, l'acide ascorbique, l'acide malique, l'acide fumarique, l'acide maléique, le bitartrate de potassium, le phosphate de sodium primaire, le citrate de sodium primaire, le tartrate de sodium primaire ou des sels acides d'aminoacides basiques ou des mélanges de ceux-ci, et les constituants basiques du mélange effervescent dégageant du CO₂ sont choisis parmi l'hydrogénocarbonate de sodium, le carbonate de sodium, le carbonate de calcium ou des mélanges de ceux-ci.

5. Préparation antiacide selon l'une des revendications 1 à 4, **caractérisée en ce que** les polymères gonflant et gélifiant fortement sont choisis parmi des polysaccharides, des dérivés de polysaccharides et des acides polyacryliques.

6. Préparation antiacide selon l'une des revendications 1 à 5, **caractérisée en ce que** la concentration des principes actifs liant les acides est de 20 à 80% en poids, celle du mélange effervescent libérant du CO₂ de 5 à 50% en poids, celle de l'agent tensio-actif polymère formant de la mousse de 0,5 à 30% en poids, et celle du polymère gonflant et gélifiant de 3 à 30% en poids, ramené au poids sec total de la préparation.

7. Préparation antiacide selon l'une des revendications 1 à 6, destinée à être utilisée comme médicament pour réguler l'hyperacidité gastrique.

8. Préparation antiacide selon la revendication 7, **caractérisée en ce que** la préparation est formulée sous la forme d'un comprimé à mâcher.

9. Procédé antiacide pour fabriquer une préparation antiacide selon l'une des revendications 1 à 8, comprenant les étapes suivantes :
(i) Mélange homogène d'un principe actif liant les acides ou de mélanges de celui-ci avec le constituant basique du mélange effervescent libérant du CO₂ ainsi que, le cas échéant, avec un ou plusieurs polymères gonflants et gélifiants,
(ii) Préparation d'une solution/suspension aqueuse contenant un ou plusieurs agents de moussage choisis parmi des poloxamères ainsi que, le cas échéant, un polymère gonflant et gélifiant ou des mélanges de polymères de ce genre,
(iii) Combinaison et mélange du mélange homogène obtenu en (i) avec la suspension/solution obtenue en (ii), granulation humide du mélange ainsi que séchage et tamisage du granulé obtenu,
(iv) Préparation d'un mélange à sec homogène à partir du composant acide du mélange effervescent dégageant du CO₂ et le cas échéant un ou plusieurs polymères gonflants et gélifiants,
(v) Incorporation du mélange obtenu en (iv) au granulé obtenu en (iii) et le cas échéant compression du granulé ainsi obtenu pour donner des comprimés,
le polymère gonflant et gélifiant étant incorporé par mélangeage homogène dans l'une au moins des étapes (i), (ii) ou (iv) et d'autres adjuvants de pastillage usuels pouvant le cas échéant être ajoutés également dans les différentes étapes du procédé.

10. Procédé selon la revendication 9, **caractérisé en ce que** les comprimés sont pressés si fortement qu'ils peuvent encore être mâchés correctement.

11. Utilisation d'une réparation antiacide selon l'une des revendications 1 à 8 ou d'une préparation antiacide qui a été préparée selon l'une des revendications 9 à 10 pour fabriquer un médicament destiné à réguler l'hyperacidité des sucs gastriques.
